# FASCICULE DE BREVET EUROPEEN

(11) **EP 0 555 160 B1**
(45) Date de publication et mention de la délivrance du brevet: **09.04.1997**
(21) Numéro de dépôt: 93420041.1
(22) Date de dépôt: 26.01.1993
(51) Int. Cl.: C07C 253/24

(54) **Procédé d'ammoxydation d'hydrocarbures saturés**
Verfahren zur Ammoxydation von gesättigten Kohlenwasserstoffen
Process for the ammoxidation of saturated hydrocarbons

(30) Priorité: 06.02.1992 FR 9201536
(43) Date de publication de la demande: 11.08.1993
(73) Titulaire: RHONE-POULENC FIBER & RESIN INTERMEDIATES, 92405 Courbevoie (FR)
(72) Inventeur: Blanchard, Gilbert, F-60330 Le Plessis Belleville (FR); Ferre, Gilbert, F-93190 Livry Gargan (FR)
(74) Mandataire: Vignally, Noel

(56) Documents cités:
- EP-A- 0 282 314
- EP-A- 0 342 777
- EP-A- 0 344 884
- J. CATAL., 127(1), 354-65 1991, AZIMOV, A. B. ET AL. '"Dehydrogenation" mechanism for ammoxidation of alkylaromatic hydrocarbons'

## Description

La présente invention concerne un procédé d'ammoxydation d'hydrocarbures saturés, c'est-à-dire la conversion d'alcanes en un mélange renfermant des nitriles α,β-insaturés.

Il est bien connu de l'homme de l'art que de très nombreuses propositions ont été formulées en ce qui concerne l'ammoxydation des oléfines et, en particulier celle du propylène. Toutefois, bien que les hydrocarbures saturés, plus largement disponibles, soient des matières premières plus intéressantes au plan économique, il est également bien connu qu'ils ne présentent pas une réactivité comparable dans ce type de réaction pour former notamment des nitriles α,β-insaturés.

L'une des difficultés rencontrées dans l'ammoxydation des hydrocarbures saturés réside dans la nécessité de pouvoir disposer de catalyseurs susceptibles de déshydrogéner l'hydrocarbure saturé dans des conditions minimisant ou supprimant la combustion de l'ammoniac et/ou celle de l'hydrocarbure, tout en assurant une sélectivité raisonnable, soit en nitrile α,β-insaturé (produit visé), par exemple en acrylonitrile au départ du propane, soit en produits valorisables (nitrile précité et oléfine), par exemple en acrylonitrile et propylène au départ du propane.

Il a déjà été proposé dans le brevet américain n° 3 365 482 d'ammoxyder notamment l'isobutane en méthacrylonitrile, sur un catalyseur à base de molybdène déposé sur de l'éta-alumine dopée par de l'antimoine, à 508°C, au départ d'un mélange gazeux renfermant de l'isobutane, de l'air, de l'ammoniac et de la vapeur d'eau (1,0/4,5/1,0/12,5) ; la sélectivité en méthacrylonitrile atteint 49 % pour un taux de conversion de l'isobutane de 22 %.

Au départ d'un mélange gazeux propane/air/ammoniac/vapeur d'eau (1,0/4,7/0,67/12,8), avec le même catalyseur et à 550°C, la sélectivité en acrylonitrile tombe à 15 % pour un taux de transformation du propane de 29 %.

Dans "Chemistry letters 1989 (pages 2173-2176)" des auteurs ont testé, pour l'ammoxydation du propane en phase vapeur, des oxydes métalliques multicomposants renfermant du molybdène et du bismuth et présentant une structure du type de celle de la scheelite. Il apparaît que, malgré les températures relativement modérées mises en oeuvre, la proportion de produits de combustion (CO, CO₂) est très élevée dans tous les cas (au moins 15 %) et que certaines compositions catalytiques testées sont très peu actives vis-à-vis de la réaction souhaitée, tout en ayant une mise en oeuvre dans des conditions situées dans le domaine d'explosivité ou très proches dudit domaine.

Il est manifeste que la coproduction de grandes quantités de CO et de CO₂ est indésirable à l'échelle industrielle.

Au surplus, l'utilisation de mélanges réactionnels se situant dans le domaine d'explosivité est d'autant moins souhaitable à l'échelle industrielle que l'on met en oeuvre le procédé en lit fixe.

Le brevet US-A-4 760 159 décrit un procédé d'ammoxydation d'un alcane ayant 3 à 5 atomes de carbone en un nitrile α,β-insaturé ayant 3 à 5 atomes de carbone, par réaction en phase gazeuse avec l'ammoniac et l'oxygène, en présence d'un catalyseur solide de formule Bᵢₐ V_{b} Lₗ Mₘ Tₜ Oₓ, dans laquelle Bi, V, M (qui est choisi parmi Mo, W, Cr, Ge et Sb) et 0 sont toujours présents.

L'observation des différents exemples de réalisation montre que les meilleures sélectivités en nitrile α,β-insaturé sont obtenues avec des catalyseurs contenant du vanadium, du bismuth et du molybdène et éventuellement un autre cation tel que le chrome, le potassium, le zinc, le césium ou l'antimoine. Le seul exemple d'utilisation d'un catalyseur à base de vanadium, de bismuth et d'antimoine de formule Bi V_{0,7} Sb_{0,5} Ox déposé à 50 % sur un mélange silice/alumine conduit à des rendements en nitrile α,β-insaturé nettement plus faibles qu'avec un catalyseur au molybdène.

En outre ce procédé conduit également à la formation d'une quantité non négligeable d'oxydes de carbone (CO +CO₂).

Il demeure donc souhaitable de disposer d'un procédé d'ammoxydation d'alcanes, permettant d'obtenir avec une sélectivité appréciable un mélange de produits valorisables renfermant un nitrile α,β-insaturé, en particulier de l'acrylonitrile, tout en diminuant les pertes de matière première par suite notamment de la formation d'oxydes de carbone.

Il serait également hautement souhaitable de disposer d'un tel procédé dans lequel le catalyseur solide serait relativement simple à préparer et actif en l'absence de promoteur halogéné et pour des mélanges de gaz ne se situant pas nécessairement dans le domaine d'explosivité.

La présente invention a donc pour objet un procédé d'ammoxydation d'alcanes en phase vapeur en présence d'un catalyseur solide comportant au moins une phase active, caractérisé en ce que la dite phase active répond à la formule empirique suivante (I) :

V Sbₐ Bi_{b} Ox (I)

dans laquelle :
- a représente un nombre entier ou fractionnaire égal ou supérieur à 1,
- b représente un nombre entier ou fractionnaire égal ou supérieur à 0,01,
- x représente un nombre entier ou fractionnaire déterminé par le degré d'oxydation des autres éléments de la phase active.

Préférentiellement dans la formule (I) de la phase active décrite précédemment :
- le symbole a représente un nombre entier ou fractionnaire jusqu'à 20 et est de préférence compris entre 1 et 10,
- le symbole b représente un nombre entier ou fractionnaire jusqu'à 20 et est de préférence compris entre 0,1 et 10.

La phase active utilisée dans le procédé de l'invention peut également comporter, outre les éléments de la formule (I), du fer et/ou du gallium et/ou de l'indium.

Cette variante de composition de la phase active peut être représentée par la formule empirique (II) :

V Sbₐ Bi_{b} M_{c} Ox (II)

dans laquelle :
- a représente un nombre entier ou fractionnaire égal ou supérieur à 1,
- b représente un nombre entier ou fractionnaire égal ou supérieur à 0,01,
- M représente un atome de fer et/ou de gallium et/ou d'indium,
- c représente un nombre entier ou fractionnaire égal ou supérieur à 0,1,
- x représente un nombre entier ou fractionnaire déterminé par le degré d'oxydation des autres éléments de la phase active.

Préférentiellement dans la formule (II) de la phase active décrite précédemment :
- le symbole a représente un nombre entier ou fractionnaire jusqu'à 20, compris entre 1 et 10,
- le symbole b représente un nombre entier ou fractionnaire jusqu'à 20, compris de préférence entre 0,1 et 10.
- le symbole c représente un nombre entier ou fractionnaire jusqu'à 20, compris de préférence entre 0,5 et 10.

Selon la présente invention des hydrocarbures saturés acycliques ayant de 3 à 12 atomes de carbone par molécule sont mis à réagir en phase vapeur avec de l'ammoniac et de l'oxygène en présence d'un catalyseur dont la phase active vient d'être précisée.

Bien entendu, dans le cadre du présent procédé, on peut utiliser des gaz diluants, inertes dans les conditions réactionnelles tels que l'hélium, l'azote et l'argon. De même, de la vapeur d'eau peut être ajoutée au mélange gazeux réactionnel dans de larges limites. Ainsi le gaz réactif (hydrocarbure saturé, ammoniac, oxygène) peut être dilué par un diluant inerte et/ou par de la vapeur d'eau. Dans cet ensemble la teneur en vapeur d'eau peut varier dans de larges limites en particulier de 0 à 50 % et, de préférence entre 3 et 30 %. Pour une bonne mise en oeuvre du procédé selon l'invention, la teneur en gaz réactif sera d'au moins 3 % et de préférence d'au moins 20 %.

Au sein du gaz réactif les teneurs respectives en hydrocarbure saturé, ammoniac et oxygène peuvent varier dans de larges limites.

La teneur en hydrocarbure saturé dans le gaz réactif est de préférence comprise entre 5 et 70 %. Celle en ammoniac est de préférence comprise entre 3 et 50 % et celle en oxygène est de préférence comprise entre 3 et 45 %.

Pour une bonne mise en oeuvre du procédé selon l'invention la composition du mélange réactif sera en dehors du domaine d'explosivité. S'agissant de l'ammoxydation du propane en l'absence de diluant inerte, la composition (propane, oxygène, ammoniac) sera avantageusement choisie à l'intérieur du quadrilatère ABDE figurant au sein du diagramme ternaire ABC représenté sur la figure 1 annexée.

Sur ce diagramme ternaire le segment AB représente la teneur en ammoniac de 100 % à 0 % ; le segment BC représente la teneur en propane de 100 à 0 % ; le segment CA représente la teneur en oxygène de 100 à 0 %. Le point D, situé sur le segment BC, correspond à une teneur en propane de 45 % ; dans le binaire (propane-O₂) ; le point E situé sur le segment AC, correspond à une teneur en ammoniac de 79 % dans le binaire (ammoniac-O₂).

Le segment DE délimite le diagramme ternaire en deux parties un triangle CDE à l'intérieur duquel se situe la zone d'explosivité (déterminée sous 1 bar et à 25°C) et un quadrilatère ABDE à l'intérieur duquel la composition du mélange gazeux réactif sera avantageusement choisie.

S'agissant de l'ammoxydation du propane en présence de gaz diluant(s) inerte(s) et/ou de vapeur d'eau, il conviendra de déterminer la composition du mélange ternaire (propane, oxygène et ammoniac) pour la situer sur le diagramme précité, lorsque le gaz diluant et/ou la vapeur d'eau est en faible proportion.

S'agissant de l'ammoxydation du propane au moyen de l'air comme source d'oxygène, la composition (propane, air et ammoniac) sera avantageusement choisie à l'intérieur du quadrilatère ABFG figurant au sein du diagramme ABC représenté sur la figure 2 annexée.

Sur ce second diagramme le segment AB représente la teneur en ammoniac de 100 % à 0 % ; le segment BC représente la teneur en propane de 100 à 0 % : le segment CA représente la teneur en air de 100 à 0 %. Le point F, situé sur le segment BC, correspond à une teneur en propane de 16 % dans le binaire (propane-air) ; le point G situé sur le segment AC, correspond à une teneur en ammoniac de 35 % dans le binaire (ammoniac-air).

Le segment FG délimite le diagramme ternaire en deux parties un triangle CFG à l'intérieur duquel se situe la zone d'explosivité (déterminée sous 1 bar à 550°C) et un quadrilatère ABFG à l'intérieur duquel la composition du mélange gazeux réactif sera avantageusement choisie.

Ce second diagramme sera utilisé dans le cas où le mélange oxygène-gaz diluant correspond à une teneur en oxygène équivalente à celle de l'air (≃ 21 % d'oxygène) ou dans le cas où ce mélange est en défaut d'oxygène, par rapport à l'air.

Au départ de propane, on obtiendra un mélange renfermant essentiellement du propylène et de l'acrylonitrile. L'acrylonitrile est un intermédiaire industriellement produit sur une large échelle, le propylène est une matière première traditionnellement utilisée pour produire de l'acrylonitrile et divers autres produits intermédiaires ien connus de l'homme de l'art.

Au départ d'isobutane, on obtiendra un mélange renfermant du méthacrylonitrile et de l'iso-butène ou des n-butènes.

Le procédé selon l'invention convient plus particulièrement à l'ammoxydation du propane.

Si l'hydrocarbure saturé mis en oeuvre peut être de qualité technique, il ne renfermera pas de quantités notables de composés à insaturation éthylénique. Ainsi le propane engagé ne renfermera de propylène qu'à l'état de traces.

Le procédé selon l'invention est réalisé sous forme de réaction en phase vapeur. En conséquence, n'importe quel dispositif convenant à la réalisation des réactions d'ammoxydation ou d'oxydation en phase vapeur peut être utilisé. Le procédé peut être conduit en continu ou en discontinu et il peut comprendre l'utilisation d'un lit fixe ou d'un lit fluidisé.

La température de réaction est en général comprise entre 300°C et 550°C et, de préférence entre 400°C et 500°C.

La pression totale du mélange réactionnel peut être supérieure ou égale à la pression atmosphérique. Elle est généralement comprise entre 1 et 6 bar et, de préférence entre 1 et 4 bar.

Le débit gazeux est fixé de telle sorte que la vitesse volumique horaire soit comprise entre 100 et 36000 h⁻¹ et, de préférence entre 200 et 20000 h⁻¹.

La vitesse volumique horaire se définit comme le rapport volume gazeux total/volume du catalyseur/heure.

Bien entendu, l'homme de l'art sera à même de trouver un compromis entre la température, le débit gazeux, la nature précise du catalyseur mise en oeuvre et les divers autres paramètres de la réaction compte-tenu de ses objectifs de production.

Dans le procédé selon l'invention, les catalyseurs peuvent être préparés ou mis en oeuvre de la façon suivante.

On synthétise d'abord une phase active constituée d'un oxyde mixte renfermant les éléments V, Sb et Bi et le cas échéant les éléments Fe et/ou Ga et/ou In.

Cette phase active peut éventuellement être déposée sur un oxyde minéral ou mélangée avec ledit oxyde minéral, connu de l'homme de l'art, comme par exemple l'alumine, la silice, la silice-alumine, la zircone, la cérine, la magnésie, l'oxyde de titane, l'oxyde de niobium, en utilisant différentes techniques connues de l'homme de l'art, telles que l'imprégnation ou le dépôt par "slurry".

La phase catalytique, constituée de la phase active seule ou de la phase active déposée sur un oxyde minéral ou mélangée avec ledit oxyde minéral, peut ensuite être mise en oeuvre sous forme massique ou à l'état particulaire ; elle peut donc être utilisée sous forme de poudre ou être mise en forme, par exemple de billes, pastilles, extrudés, particules concassées, selon différentes techniques connues.

Pour une mise en oeuvre du procédé en lit fixe, ces techniques peuvent être, par exemple, le pastillage, l'enrobage sur un support inerte ou sur substrat céramique ou métallique de type monolithique.

Pour une mise en oeuvre du procédé en lit mobile ou en lit fluidisé, la phase catalytique est généralement mise en forme par atomisation, séchage et calcination.

La phase catalytique ainsi mise en forme ou sous forme de poudre constitue le catalyseur selon l'invention.

Lorsque la phase catalytique comprend la phase active déposée sur un oxyde minéral ou mélangée avec ledit oxyde minéral, une variante de préparation peut consister à réaliser en une seule étape, la synthèse de la phase active et son dépôt sur l'oxyde minéral ou son mélange avec ledit oxyde minéral.

Dans ce qui suit la synthèse de la phase active et son dépôt sur l'oxyde minéral ou son mélange avec ledit oxyde minéral seront d'abord décrits séparément, mais la description portera également sur la variante de synthèse de la phase active en présence de l'oxyde minéral.

La préparation des phases actives mises en oeuvre dans le procédé selon l'invention peut être réalisée par diverses techniques connues, telles que le mélange de sels ou d'oxydes convenables des différents métaux dans l'eau ou dans un autre solvant, suivi de l'évaporation jusqu'à siccité, ou de la précipitation par ajout d'une base telle que l'ammoniaque ou d'un acide tel que l'acide chlorhydrique, ou l'atomisation d'une suspension obtenue après mélange des sels convenables.

Les sels convenables les plus couramment employés contiennent des anions et des cations qui peuvent, par exemple, être décomposés par la chaleur lors des étapes ultérieures.

Comme exemples de sels ou oxydes convenables de vanadium, on peut citer : le vanadate d'ammonium, les oxyhalogénures de vanadium tels que VOCl₃, VOCl₂, (VO₂)Cl, VOCl, VOBr, VOBr₂, VOBr₃, VOF₃, VOF₂, les halogénures de vanadium tels que VF₃, VBr₃, VCl₂, VCl₃, VCl₄, VF₅, VF₄, VBr₂, VI₂, le sulfate de vanadyle, l'acétylacétonate de vanadyle, l'acide méta-vanadique, l'hexacarbonyle de vanadium, le tri-isopropoxyde d'oxyde de vanadium, les oxydes de vanadium tels que V₂O₅, V₇O₁₃, VO, VO₂, V₂O₃, V₃O₇.

Comme exemples de sels ou oxydes convenables d'antimoine, on peut citer l'oxychlorure d'antimoine, les halogénures d'antimoine tels que SbBr₃, SbCl₃, SbF₃, SbI₃, SbCl₅, SbF₅, SbI₅, le sulfate d'antimoine, l'acétate d'antimoine, le tartrate d'antimoine, l'éthoxyde d'antimoine, le butoxyde d'antimoine, l'éthylèneglycoxyde d'antimoine, l'oxysulfate d'antimoine, les oxydes d'antimoine tels que Sb₂O₃, Sb₂O₄, Sb₂O₅.

Comme exemples de sels ou oxydes convenables de bismuth, on peut citer : le nitrate de bismuth, les oxyhalogénures de bismuth tels que BiOCl, BiOI, BiOBr, BiOF, les halogénures de bismuth tels que BiCl₃, BiBr₃, BiF₃, BiI₃, BiCl₄, BiI₂, le sulfate de bismuth, l'acétate de bismuth, le propionate de bismuth, l'oxalate de bismuth, le citrate de bismuth, le tartrate de bismuth, le lactate de bismuth, le benzoate de bismuth, le salicylate de bismuth, l'oxycarbonate de bismuth, l'oxynitrate de bismuth, l'oxyperchlorate de bismuth, les oxydes de bismuth tels que Bi₂O₃, Bi₂O₅.

Comme exemples de sels ou oxydes convenables de fer, on peut citer le nitrate de fer, le perchlorate de fer, l'oxychlorure de fer, les halogénures de fer tels que FeCl₃, FeCl₂, FeBr₃, FeBr₂, FeF₃, FeF₂, FeI₂, le phosphate de fer, le sulfate de fer, l'iodate de fer, le fer-pentacarbonyle, l'acétate de fer, l'acétylacétonate de fer, le citrate de fer, le formiate de fer, le gluconate de fer, le glycéro-phosphate de fer, le lactate de fer, le malate de fer, le méthoxyde de fer, l'oléate de fer, l'oxalate de fer, le tartrate de fer, le 2-éthyl-hexanoate de fer, les oxydes de fer tels que Fe₂O₃, Fe₃O₄, FeO.

Comme exemples de sels ou oxydes convenables de gallium, on peut citer le nitrate de gallium, le perchlorate de gallium, l'oxychlorure de gallium, les halogénures de gallium tels que GaCl₃, GaCl₂, GaBr₃, GaF₃, GaI₃, le sulfate de gallium, l'acétate de gallium, l'acétylacétonate de gallium, l'oxalate de gallium, les oxydes de gallium tels que Ga₂O₃, Ga₂O.

Comme exemples de sels ou oxydes convenables d'indium, on peut citer le nitrate d'indium, le perchlorate d'indium, les halogénures d'indium tels que InCl₃, InCl₂, InCl, InBr₃, InBr₂, InBr, InF₃, InI₃, InI₂, InI, le phosphate d'indium, le sulfate d'indium, l'iodate d'indium, l'acétate d'indium, l'acétylacétonate d'indium, le méthoxyde d'indium, les oxydes d'indium tels que In₂O₃, In₂O, InO.

La synthèse de la phase active est généralement réalisée par la méthode dite d'évaporation, de la façon suivante : on prépare une suspension aqueuse des sels ou oxydes convenables, on évapore en chauffant entre 20 et 100°C jusqu'à l'obtention d'une pâte visqueuse que l'on sèche. Le précurseur ainsi obtenu peut ensuite être broyé et calciné entre 200 et 1000°C. La phase active ainsi obtenue après refroidissement peut ensuite être broyée pour que sa granulométrie n'excède pas 400 µm environ.

Le précurseur peut aussi être obtenu selon une variante comprenant la précipitation avec addition, par exemple, d'ammoniaque ou d'acide chlorhydrique, au cours ou en fin de mélange des sels ou oxydes. Il est préférable de chauffer la suspension entre 20 et 100°C pour parfaire la précipitation des espèces.

La suspension obtenue peut être évaporée selon les conditions décrites ci-avant, ou filtrée et lavée. La pâte ou le gâteau de filtration, obtenus respectivement par évaporation ou par filtration, sont ensuite séchés, broyés et calcinés selon les conditions décrites ci-avant dans le cadre de la méthode d'évaporation, pour donner la phase active.

Cette phase active peut éventuellement être déposée sur un ou plusieurs oxydes minéraux ou être mélangée avec le ou lesdits oxydes minéraux, connus de l'homme de l'art. A titre d'exemples d'oxydes minéraux susceptibles de convenir à la préparation de catalyseurs utilisables dans le cadre du procédé selon l'invention, on peut citer de manière non limitative l'alumine, la silice, la silice-alumine, la zircone, la cérine, la magnésie, l'oxyde de titane, l'oxyde de niobium.

Le dépôt sur ces oxydes ou le mélange avec ces oxydes minéraux peuvent s'effectuer par différentes techniques connues, comme par exemple l'imprégnation ou le dépôt par "slurry".

La quantité de phase active, qui peut varier dans de larges limites, est en pratique comprise entre 5 et 100 % et de préférence entre 10 et 50 % en poids par rapport à l'ensemble phase active + oxyde minéral.

Une autre méthode couramment utilisée dans le cadre de l'invention est le mélange de l'oxyde minéral avec les sels ou oxydes convenables des différents métaux de la phase active selon les conditions décrites ci-avant dans le cadre de la préparation des phases actives. Une fois ce mélange réalisé, le précurseur peut être obtenu par les méthodes dites d'évaporation ou de précipitation, puis la pâte ou le gâteau de filtration obtenus sont séchés, broyés et calcinés selon les conditions décrites ci-avant dans le cadre de la préparation des phases actives.

La phase active seule ou déposée sur un oxyde minéral ou mélangée avec un oxyde minéral tels que décrits précédemmment, constituent la phase catalytique.

Les phases catalytiques en cause peuvent être mises en oeuvre sous forme massique ou à l'état particulaire. Ces phases peuvent donc être utilisées sous forme de poudre ou être mises, par exemple, sous forme de billes, pastilles, extrudés, particules concassées, selon différentes techniques connues.

Pour une mise en oeuvre du procédé en lit fixe, on peut citer à titre d'exemples de techniques pouvant convenir à la préparation des catalyseurs utilisables dans le cadre du procédé selon l'invention : le pastillage, l'enrobage sur un support inerte ou sur un substrat céramique ou métallique de type monolithique.

Les phases catalytiques selon l'invention peuvent par exemple être mises en forme par compression, de façon à obtenir des pastilles. Ces pastilles peuvent ensuite être éventuellement concassées en éclats. Les valeurs précises de la pression, du diamètre et de l'épaisseur des pastilles, et de la granulométrie des éclats pourront être choisies par l'homme de l'art en fonction de la perte de charge admissible dans le réacteur.

Les phases catalytiques selon l'invention peuvent également être déposées sur un support inerte ou l'enrober. La nature de ce support n'est pas critique dès lors qu'il est chimiquement inerte vis-à-vis des réactifs dans les conditions réactionnelles choisies. A titre d'exemples de supports susceptibles de convenir à la préparation de catalyseurs utilisables dans le cadre du procédé selon l'invention, on peut citer : la silice, l'alumine, la silice-alumine, l'argile frittée, le carborandum, la magnésie, le silicate de magnésium, la terre de diatomée. Ce support est de préférence non poreux et peut notamment être à base d'oxyde réfractaire sous forme particulaire, le support le plus couramment employé étant à base d'argile. Ce support peut par exemple être constitué de billes d'argile, inertes, pleines, solides et rugueuses, de diamètre compris entre 0.5 et 6 mm. La valeur précise du diamètre des billes pourra être choisie en fonction de la perte de charge admissible dans le réacteur. Ce support peut également être rendu non poreux par émaillage.

Ce support peut également être un substrat céramique, ledit substrat étant de préférence sous forme d'une structure inerte et rigide de type monolithique comprenant des canaux ou conduits. De tels supports sont bien connus et ont été largement décrits dans la littérature. Les substrats en matières céramiques utilisés sont notamment ceux comprenant comme matière principale la cordiérite, l'alumine, la mullite, la porcelaine, les carbures de bore ou de silicium.

Ce support peut également être un substrat métallique. De tels supports sont bien connus. Les substrats métalliques convenables sont notamment ceux obtenus à partir d'alliages de fer, de nickel et de chrome, ou ceux obtenus à partir d'alliages de fer, de chrome, aluminium et cobalt tels que ceux connus sous la marque KANTHAL ou ceux obtenus à partir d'alliages de fer, de chrome, d'aluminium et d'yttrium connus sous la marque FECRALLOY. Le métal peut aussi être de l'acier carboné ou de la fonte simple.

Lorsqu'on fait appel à un catalyseur enrobé, la quantité de phase catalytique qui peut varier dans de larges limites, est en pratique comprise entre 1 et 50 % et de préférence entre 5 et 35 % en poids par rapport à l'ensemble support + phase catalytique.

Ainsi, certains catalyseurs, utiles pour une mise en oeuvre du procédé en lit fixe, peuvent être obtenus par enrobage de manière connue en soi, des phases catalytiques, intermédiaires ou finies, broyées. Cette méthode classique consiste à déposer autour de billes inertes, mais rugueuses, une couche de phase catalytique intermédiaire ou finie. Une fois les billes recouvertes de la quantité voulue de la phase catalytique, elles sont séchées par de l'air chaud, entre 70 et 150°C pendant au moins 30 minutes, puis introduites dans un four pour être calcinées entre 300 et 600°C de préférence entre 450 et 500°C, pendant au moins 3 heures.

Certains catalyseurs utiles pour une mise en oeuvre du procédé selon l'invention en lit mobile ou en lit fluidisé peuvent être obtenus par la technique connue en soi du séchage par atomisation en atmosphère, de préférence non réductrice. Par une telle opération, le cas échéant suivie d'une calcination à une température de l'ordre de 400 à 1100°C, on obtient des poudres de forme sphérique de diamètre compris entre 5 et 700 µm. Des poudres constituées pour au moins 80 % en poids de particules dont la dimension est comprise entre 5 et 200 µm sont préférées dans le cadre d'une utilisation en lit fluidisé.

La phase catalytique ainsi mise en oeuvre sous forme massique ou à l'état particulaire, constitue le catalyseur selon l'invention.

Les produits de la réaction peuvent être récupérés dans les gaz effluents par tout moyen approprié. Par exemple les gaz effluents peuvent passer dans un condenseur contenant de l'acide sulfurique dilué pour neutraliser l'ammoniac non converti. Les gaz peuvent ensuite passer sur une colonne absorbante réfrigérée pour condenser l'acrylonitrile, l'acétonitrile et l'acide cyanhydrique, les vapeurs non condensées contenant principalement du propane non converti, du propylène, des hydrocarbures légers et le cas échéant du CO₂. On peut ensuite séparer l'acrylonitrile et l'acide cyanhydrique de l'acétonitrile par distillation, puis distiller à son tour le mélange acrylonitrile-acide cyanhydrique récupéré pour séparer l'acrylonitrile de l'acide cyanhydrique.

Les exemples ci-après illustrent la présente invention.

### EXEMPLE 1 - Préparation du catalyseur (A₁) selon l'invention, de formule empirique (III) suivante : V Sb_{3,5}Bi₂Ox/Al₂O₃ (25/75 % en poids) (III)

a) On prépare une solution (a) de vanadate d'ammonium par dissolution de 2,34 g de NH₄VO₃ dans 400 cm³ d'eau permutée, une solution (b) de nitrate de bismuth par dissolution de 19,4 g de Bi (NO₃)₃, 5 H₂O dans 50 cm³ d'eau permutée et 15 cm³ d'acide nitrique concentré et une solution (c) de chlorure d'antimoine par dissolution de 10,2 g de Sb₂O₃ dans 250 cm³ d'acide chlorhydrique 1 N. On ajoute, sous agitation à 70°C environ, la solution (b) à la solution (a), puis on ajoute la solution (c). On ajoute ensuite, à 90°C environ, 47,3 g de Al₂O₃. On chauffe à 105°C pendant 6 h, on évapore à sec, on sèche à 120°C pendant 15 h environ, puis on calcine à 550°C pendant 10 h.
b) Le produit de formule (III) ainsi obtenu est ensuite comprimé sous une pression de 4300 kg/cm². On obtient ainsi des pastilles de 3 cm de diamètre et d'environ 0,5 cm d'épaisseur. Ces pastilles sont concassées en éclats de granulométrie comprise entre 0,3 et 0,8 cm constituant le catalyseur A₁ conforme à l'invention.

### EXEMPLE 2 - Préparation du catalyseur (A₂) selon l'invention, de formule empirique suivante : V Sb_{3,5}Bi₂ Ox/Al₂O₃ (25/75 % en poids) enrobé sur argile

Le produit de formule (III) obtenu dans l'étape a) de l'exemple 1 est ensuite utilisé de la manière suivante.

On saupoudre lentement 15 g dudit produit préparé précédemment sur 100 g de support inerte composé de billes d'argile de diamètre moyen 4,8 mm, préalablement placées dans un drageoir en rotation et humidifiées par du glucose en solution aqueuse à 10 % ; dès que les billes sont sèches à l'extérieur, on pulvérise une petite quantité de la solution de glucose. Puis on saupoudre à nouveau le produit de formule (III) sur les billes. Ces opérations sont poursuivies alternativement jusqu'à enrobage de la totalité du produit de formule (III).

On sèche ensuite à 120°C pendant 2 heures et on effectue une calcination de 6 heures à 480°C.

Le catalyseur A₂ ainsi obtenu est constitué de 8,8 % en poids de V Sb_{3,5} Bi₂ Ox/Al₂O₃ (25/75 % en poids) enrobé sur billes d'argile.

### EXEMPLE 3 - Préparation du catayseur (B) selon l'invention, de formule empirique (IV) suivante : V Sb_{1,64} Bi_{0,9} Ox, enrobé sur billes d'argile

On prépare un produit de formule (IV) V Sb_{1,64} BiO_{0,9} Ox selon le protocole opératoire suivant : on prépare une suspension de 19,3 g de V₂O₅ dans 400 cm³ d'eau permutée ; on ajoute goutte à goutte, sous agitation, 122,5 g de H₂O₂ en solution à 30 % dans l'eau, en trois parties respectivement de 49 g, 49 g et 24,5 g. On ajoute ensuite une solution constituée de 92,7 g de Bi(NO₃)₃, 5 H₂O dans 24 cm³ de HNO₃ concentré et 61 cm³ d'eau permutée. Puis on ajoute 50,8 g de Sb₂ O₃. On porte à reflux, on laisse évaporer à sec, on sèche à 120°C pendant 15 h environ, puis on calcine à 650°C pendant 8 h.

Le produit de formule (IV) ainsi obtenu présente une surface spécifique mesurée selon la méthode B.E.T. de 11 m²/g.

9 g du produit de formule (IV) ainsi préparé sont saupoudrés lentement sur 66 g de support inerte, composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit de formule (IV) sur les billes. On poursuit ces opérations alternativement jusqu'à enrobage de la totalité dudit produit. On sèche ensuite à 120°C pendant 2 h et on calcine à 480°C pendant 6 h.

Le catalyseur (B) ainsi obtenu, conforme à l'invention, est constitué de 12 % en poids de V Sb_{1,64} Bi_{0,9} Ox enrobés sur billes d'argile.

### EXEMPLE 4 - Préparation du catalyseur (G) selon l'invention, de formule empirique (V) suivante : V Sb_{2,5} Bi_{0,5}Ox/Al₂O₃ (50/50 % en poids)

On prépare un produit de formule (V) V Sb_{2,5} Bi_{0,5} Ox selon le protocole opératoire suivant.

On ajoute sous agitation 37,75 g de Sb₂O₅ à une solution chaude de 10,92 g de vanadate d'ammonium dans 300 cm³ d'eau permutée. On ajoute ensuite une solution de nitrate de bismuth consistant en 22,6 g de Bi(NO₃)₃, 5H₂O, 58 cm³ d'eau permutée et 8,7 cm³ d'acide nitrique à 65 %.

On ajoute ensuite 57 g de Al₂O₃. On évapore à sec, on sèche le produit obtenu à 120°C pendant environ 15 h, puis on le calcine à 290°C pendant 3 heures, à 425°C pendant 3 h et à 610°C pendant 3 h.

Le produit obtenu est ensuite comprimé sous une pression de 4300 kg/cm². On obtient ainsi des pastilles de 3 cm de diamètre et d'environ 0,5 cm d'épaisseur. Ces pastilles sont ensuite concassées en éclats de granulométrie comprise entre 0,3 cm et 0,8 cm.

Le catalyseur (G) ainsi obtenu, conforme à l'invention, est constitué de V Sb_{2,5} Bi_{0,5} Ox/Al₂O₃ à 50/50 % en poids.

### EXEMPLE 5 - Préparation du catalyseur (H) selon l'invention, de formule empirique (VI) suivante : V Sb_{3,5} Bi₂ Fe₂ Ox/Al₂O₃ (25/75 % en poids)

On prépare un produit de formule (VI) V Sb_{3,5} Bi₂ Fe₂ Ox selon le protocole opératoire suivant.

On prépare une solution de vanadate d'ammonium par dissolution de 2,34 g de NH₄ VO₃ dans 350 cm³ d'eau permutée. On ajoute sous agitation et à 70°C environ, une solution de nitrate de bismuth préparée par dissolution de 19,4 g de Bi(NO₃)₃,5H₂O dans 50 cm³ d'eau permutée et 15 cm³ d'acide nitrique concentré, puis une solution de nitrate de fer préparée par dissolution de 16,2 g de Fe(NO₃)₃,9H₂O dans 70 cm³ d'eau permutée. On ajoute ensuite une suspension chaude de 10,2 g de Sb₂O₃ dans 210 cm³ d'eau permutée et 21 cm³ d'acide chlorhydrique concentré, puis 47,3 g d'alumine lorsque le mélange est à une température d'environ 95°C.

On porte à reflux pendant 6 h, puis on évapore à sec, on sèche à 120°C pendant 15 h environ et on calcine à 550°C pendant 10 h environ.

Le produit obtenu est ensuite comprimé sous une pression de 4300 kg/cm². On obtient ainsi des pastilles de 3 cm de diamètre et d'environ 0,5 cm d'épaisseur. Ces pastilles sont ensuite concassées en éclats de granulométrie comprise entre 0,3 cm et 0,8 cm.

Le catalyseur (H) ainsi obtenu, conforme à l'invention, est constitué de V Sb_{3,5} Bi₂ Fe₂ Ox/Al₂O₃ à 25/75 % en poids.

### EXEMPLE 6- Préparation du catalyseur (J) selon l'invention, de formule empirique (VII) suivante : V Sb₅ Bi₂ Fe₂ Ox/Al₂O₃ (25/75 % en poids)

On prépare un produit de formule (VII) V Sb₅ Bi₂ Fe₂ Ox selon le protocole opératoire suivant.

On prépare une solution de vanadate d'ammonium par dissolution de 2,34 g de NH₄ VO₃ dans 350 cm³ d'eau permutée. On ajoute sous agitation et à 70°C environ, une solution de nitrate de bismuth préparée par dissolution de 19,4 g de Bi(NO₃)₃,5H₂O dans 50 cm³ d'eau permutée et 15 cm³ d'acide nitrique concentré, puis une solution de nitrate de fer préparée par dissolution de 16,2 g de Fe(NO₃)₃,9H₂O dans 70 cm³ d'eau permutée. On ajoute ensuite une suspension chaude de 14,57 g de Sb₂O₃ dans 300 cm³ d'eau permutée et 30 cm³ d'acide chlorhydrique concentré, puis 47,3 g d'alumine lorsque le mélange est à une température d'environ 90°C.

On porte à reflux pendant 6 h, puis on évapore à sec, on sèche à 120°C pendant 15 h environ et on calcine à 550°C pendant 10 h environ.

Le produit obtenu est ensuite comprimé sous une pression de 4300 kg/cm². On obtient ainsi des pastilles de 3 cm de diamètre et d'environ 0,5 cm d'épaisseur. Ces pastilles sont ensuite concassées en éclats de granulométrie comprise entre 0,3 cm et 0,8 cm.

Le catalyseur (J) ainsi obtenu, conforme à l'invention, est constitué de V Sb₅ Bi₂ Fe₂ Ox/Al₂O₃ à 25/75 % en poids.

### EXEMPLE 7 - Préparation du catalyseur (K) selon l'invention, de formule empirique (VIII) suivante : V Sb₅ Bi_{0,5} Fe₅ Ox/Al₂O₃ (25/75 % en poids)

On prépare un produit de formule (VIII) V Sb₅ Bi_{0,5} Fe₅ Ox selon le protocole opératoire suivant.

On prépare une solution de vanadate d'ammonium par dissolution de 2,34 g de NH₄ VO₃ dans 350 cm³ d'eau permutée. On ajoute sous agitation et à 70°C environ, une solution de nitrate de bismuth préparée par dissolution de 4,85 g de Bi(NO₃)₃,5H₂O dans 20 cm³ d'eau permutée et 3,75 cm³ d'acide nitrique concentré, puis une solution de nitrate de fer préparée par dissolution de 40,5 g de Fe(NO₃)₃,9H₂O dans 100 cm³ d'eau permutée. On ajoute ensuite une suspension chaude de 14,57 g de Sb₂O₃ dans 300 cm³ d'eau permutée et 30 cm³ d'acide chlorhydrique concentré, puis 47,3 g d'alumine lorsque le mélange est à une température d'environ 90°C.

On porte à reflux pendant 6 h, puis on évapore à sec, on sèche à 120°C pendant 15 h environ et on calcine à 550°C pendant 10 h environ.

Le produit obtenu est ensuite comprimé sous une pression de 4300 kg/cm². On obtient ainsi des pastilles de 3 cm de diamètre et d'environ 0,5 cm d'épaisseur. Ces pastilles sont ensuite concassées en éclats de granulométrie comprise entre 0,3 cm et 0,8 cm.

Le catalyseur (K) ainsi obtenu, conforme à l'invention, est constitué de V Sb₅ Bi_{0,5} Fe₅ Ox/Al₂O₃ à 25/75 % en poids.

### EXEMPLE 8 - Préparation du catalyseur (L) selon l'invention, de formule empirique (IX) suivante : V Sb₆ Bi₆Fe Ox/Al₂O₃ (25/75 % en poids)

On prépare un produit de formule (IX) V Sb₆ Bi₆ Fe Ox selon le protocole opératoire suivant.

On prépare une solution de vanadate d'ammonium par dissolution de 2,34 g de NH₄ VO₃ dans 350 cm³ d'eau permutée. On ajoute sous agitation et à 70°C environ, une solution de nitrate de bismuth préparée par dissolution de 58,2 g de Bi(NO₃)₃,5H₂O dans 120 cm³ d'eau permutée et 45 cm³ d'acide nitrique concentré, puis une solution de nitrate de fer préparée par dissolution de 8,1 g de Fe(NO₃)₃,9H₂O dans 20 cm³ d'eau permutée. On ajoute ensuite une suspension chaude de 17,5 g de Sb₂O₃ dans 180 cm³ d'eau permutée et 36 cm³ d'acide chlorhydrique concentré, puis 47,3 g d'alumine lorsque le mélange est à une température d'environ 90°C.

On porte à reflux pendant 6 h, puis on évapore à sec, on sèche à 120°C pendant 15 h environ et on calcine à 550°C pendant 10 h environ.

Le produit obtenu est ensuite comprimé sous une pression de 4300 kg/cm². On obtient ainsi des pastilles de 3 cm de diamètre et d'environ 0,5 cm d'épaisseur. Ces pastilles sont ensuite concassées en éclats de granulométrie comprise entre 0,3 cm et 0,8 cm.

Le catalyseur (L) ainsi obtenu, conforme à l'invention, est constitué de V Sb₆ Bi₆ Fe Ox/Al₂O₃ à 25/75 % en poids.

### ESSAI COMPARATIF 1 - Préparation du catalyseur (C) non conforme à l'invention, de formule empirique suivante : V Sb_{1,64} Ox, enrobé sur billes d'argile

On prépare un produit de composition V Sb_{1,64} Bi_{0,9} Ox selon le protocole opératoire suivant. On prépare une suspension de 27,58 g de V₂O₅ dans 400 cm³ d'eau permutée ; on ajoute goutte à goutte, sous agitation, 175 g de H₂O₂ en solution à 30 % dans l'eau, en trois parties respectivement de 70 g, 70 g et 35 g. On ajoute ensuite 72,56 g de Sb₂O₃, puis 200 cm³ d'eau permutée. On porte à reflux et on laisse évaporer à sec. Le produit obtenu est ensuite séché à 120°C pendant 15 h environ, puis calciné à 650°C pendant 8 h.

Le produit ainsi obtenu présente une surface spécifique mesurée selon la méthode B.E.T. de 55 m²/g.

7,5 g du produit V Sb_{1,64} Ox ainsi obtenu sont saupoudrés lentement sur 50 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit V Sb_{1,64} Ox sur les billes. On poursuit ces opérations alternativement jusqu'à enrobage de la totalité dudit produit. On sèche ensuite à 120°C pendant 2 h et on calcine à 480°C pendant 6 h.

Le catalyseur (C) ainsi obtenu, non conforme à l'invention, est constitué de 10,55 % en poids de V Sb_{1,64} Ox enrobés sur billes d'argile.

### ESSAI COMPARATIF 2 - Préparation du catalyseur (D) non conforme à l'invention, de formule empirique suivante : V Sb_{3,5} Ox/Al₂ O₃ (25/75 % en poids)

On prépare un produit de composition V Sb_{3,5} Ox/Al₂ O₃ (25/75 % en poids) selon le protocole opératoire suivant. On prépare une solution (a) de vanadate d'ammonium par dissolution de 2,34 g de NH₄VO₃ dans 400 cm³ d'eau permutée et une suspension (b) de chlorure d'antimoine par dissolution de 10,2 g de Sb₂O₃ dans 21 cm³ d'acide chlorhydrique et 230 cm³ permutée. On ajoute sous agitation à 80-90°C environ, la suspension (b) à la solution (a), puis 47,3 g de Al₂O₃. On maintient à cette température pendant 6 h, on évapore à sec, on séche à 120°C pendant 15 h environ, puis on calcine à 550°C pendant 10 h.

Ce produit est ensuite comprimé sous une pression de 4300 kg/cm². On obtient ainsi des pastilles de 3 cm de diamètre et environ 0,5 cm d'épaisseur. Ces pastilles sont ensuite concassées en éclats de granulométrie comprise entre 0,3 et 0,8 cm, constituant le catalyseur (D), de composition V Sb_{3,5} Ox/Al₂ O₃ (25/75 % en poids), non conforme à l'invention.

### ESSAI COMPARATIF 3 - Préparation du catalyseur (E) non conforme à l'invention, de formule empirique suivante : Bi V_{0,7} Sb_{0,5} Ox/SiO₂/Al₂ O₃ (50/25/25 % en poids)

On prépare un produit de composition Bi V_{0,7} Sb_{0,5} Ox/SiO₂/Al₂ O₃ (50/25/25 % en poids) selon le protocole opératoire décrit dans le brevet US 4 760 159.

On ajoute, sous agitation, 10,78 g de Sb₂O₅ à une solution chaude de vanadate d'ammonium (10,92 g de NH₄Vo₃ dans 300 cm³ d'eau permutée). On ajoute ensuite une solution de nitrate de bismuth (64,7 g de Bi(NO₃)₃, 5 H₂O dans 150 cm³ d'eau permutée et 25 cm³ d'acide nitrique à 65 %). On ajoute ensuite 25 g de Al₂O₃ et 25 g de SiO₂. On laisse évaporer à sec, on sèche le produit obtenu à 125°C pendant environ 15 h, puis on le calcine à 290°C pendant 3 h, à 425°C pendant 3 h et à 610°C pendant 3 h.

Le produit obtenu est ensuite comprimé sous une pression de 4300 kg/cm². On obtient ainsi des pastilles de 3 cm de diamètre et environ 0,5 cm d'épaisseur. Ces pastilles sont ensuite concassées en éclats de granulométrie comprise entre 0,3 et 0,8 cm, constituant le catalyseur (E), de composition Bi V_{0,7} Sb_{0,5} Ox/SiO₂/Al₂ O₃ (50/25/25 % en poids), non conforme à l'invention.

### ESSAI COMPARATIF 4 - Préparation du catalyseur (F) non conforme à l'invention, de formule empirique suivante : Bi V_{0,7} Sb_{0,5} Ox/Al₂ O₃ (50/50% en poids)

On prépare un produit de composition Bi V_{0,7} Sb_{0,5} Ox/Al₂ O₃ (50/50 % en poids) selon le protocole opératoire décrit dans le brevet US 4 760 159.

On ajoute sous agitation, 10,78 g de Sb₂O₅ à une solution chaude de vanadate d'ammonium (10,92 g de NH₄Vo₃ dans 300 cm³ d'eau permutée). On ajoute ensuite une solution de nitrate de bismuth constituée de 64,7 g de Bi(NO₃)₃, 5H₂O, 150 cm³ d'eau permutée et 25 cm³ d'acide nitrique à 65 %. On ajoute ensuite 50 g de Al₂O₃. On laisse évaporer à sec, on sèche le produit obtenu à 120°C pendant environ 15 h, puis on le calcine à 290°C pendant 3 h, à 425°C pendant 3 h et à 610°C pendant 3 h.

Le produit obtenu est ensuite comprimé sous une pression de 4300 kg/cm². On obtient ainsi des pastilles de 3 cm de diamètre et d'environ 0,5 cm d'épaisseur. Ces pastilles sont ensuite concassées en éclats de granulométrie comprise entre 0,3 et 0,8 cm, constituant le catalyseur (F), de composition Bi V_{0,7} Sb_{0,5} Ox/Al₂ O₃ (50/50 % en poids), non conforme à l'invention.

### ESSAI COMPARATIF 5 - Préparation du catalyseur (M) non conforme à l'invention, de formule empirique suivante : V Sb_{3,5} Fe₂ Ox/Al₂O₃ (25/75 % en poids)

a) On prépare une solution (a) de vanadate d'ammonium par dissolution de 2,34 g de NH₄VO₃ dans 400 cm³ d'eau permutée, une solution (b) de nitrate de fer par dissolution de 16,2 g de Fe (NO₃)₃, 9 H₂O dans 70 cm³ d'eau permutée et une suspension (c) de chlorure d'antimoine par dissolution de 10,2 g de Sb₂O₃ dans 250 cm³ d'acide chlorhydrique 1 N. On ajoute sous agitation, à 70°C environ, la solution (b) à la solution (a), puis on ajoute la suspension (c). On ajoute ensuite, à 90°C environ, 47,3 g de Al₂O₃. On maintient à 90°C pendant 6 h, on évapore à sec, on sèche à 120°C pendant 15 h environ, puis on calcine à 550°C pendant 10 h.
b) Le produit ainsi obtenu est ensuite comprimé sous une pression de 4300 kg/cm². On obtient ainsi des pastilles de 3 cm de diamètre et d'environ 0,5 cm d'épaisseur. Ces pastilles sont concassées en éclats de granulométrie comprise entre 0,3 et 0,8 cm constituant le catalyseur M non conforme à l'invention.

### MODE OPERATOIRE GENERAL DES TESTS D'AMMOXYDATION

L'échantillon de catalyseur est préalablement porté sur un banc de mesure à la température de 150°C sous balayage d'hélium pendant 10 min, puis il est soumis à un flux gazeux dont la composition sera précisée pour chaque exemple et qui renferme du propane, de l'ammoniac, de l'oxygène, de la vapeur d'eau et de l'hélium.

La pression totale du mélange réactionnel comprise entre 1 et 6 bar, sera également précisée pour chaque exemple.

Le débit total gazeux est défini de façon à avoir une vitesse volumique horaire (VVH) comprise entre 100 et 36000 h⁻¹, dont la valeur précise sera indiquée pour chaque exemple.

Volume de catalyseur :
(phase catalytique + support éventuel) : 25 cm³.
Le principe du test d'ammoxydation du propane est le suivant :
- On porte le catalyseur à une température T₁, par exemple 300°C, et après 30 min de stabilisation à la température T₁, on détermine par chromatographie en phase gazeuse la composition du mélange en sortie de réacteur.
- On calcule les pourcentages de conversion et les sélectivités obtenus sur le catalyseur examiné à la température d'entrée T1 par des relations du type : conversion du propane (en moles %) = propane transformé/propane introduit sélectivité en acrylonitrile (en moles %) = propane transformé en acrylonitrile/propane converti
- On porte ensuite le catalyseur de 300 à 550°C par incrément de 20°C et on détermine toutes les 40 min les pourcentages de conversion et les sélectivités.

Dans les exemples ci-après, les conventions suivantes sont utilisées :
- TTC₃H8 =: conversion du propane
- SACN =: sélectivité en acrylonitrile
- SACN+C₃H₆ =: sélectivité en acrylonitrile et propylène
- SCOX =: sélectivité en monoxyde et dioxyde de carbone
- SAMMOX =: sélectivité en acétonitrile, en acide cyanhydrique et autres sous-produits d'(amm)oxydation
- SC₁C₂ =: sélectivité en méthane, éthane et éthylène.

### EXEMPLE 9 ET ESSAI COHPARATIF 6

Mesure des performances des catalyseurs (A₁) et(E).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 25 %
   NH₃ = 25 %
   O₂ = 10 %
   H₂O = 20 %
   He = 20 %

Les conditions de température et les résultats obtenus sont rassemblés dans le tableau 1 ci-après.

**TABLEAU 1**

| Essais | Exemple 9 | | | | Essai comparatif 6 | | | |
|---|---|---|---|---|---|---|---|---|
| Catalyseur utilisé | A₁ | | | | E | | | |
| Température (en°C) | 400 | 420 | 440 | 460 | 400 | 420 | 440 | 460 |
| TTC₃H₈ % | 4 | 6 | 9 | 13 | 5 | 6 | 6 | 5 |
| SACN % | 37 | 49 | 60 | 67 | 22 | 27 | 27 | 19 |
| SACN + C₃H₆ % | 58 | 70 | 77 | 79 | 65 | 66 | 68 | 66 |
| SAMMOX % | 42 | 30,5 | 23 | 21 | 35 | 31 | 27 | 29 |
| SC₁C₂ % | 0 | 0 | 0 | 0 | 0 | 3 | 5 | 5 |
| SCOX % | 0 | 0 | 0 | 0 | 0 | 0 | 0 | 0 |

### EXEMPLE 10 ET ESSAIS COMPARATIFS 7 ET 8

Mesure des performances des catalyseurs (A₁), (D) et (E).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 48 %
   NH₃ = 9 %
   O₂ = 18 %
   H₂O = 20 %
   He = 5 %

Les conditions de température et les résultats obtenus sont rassemblés dans le tableau 2 ci-après.

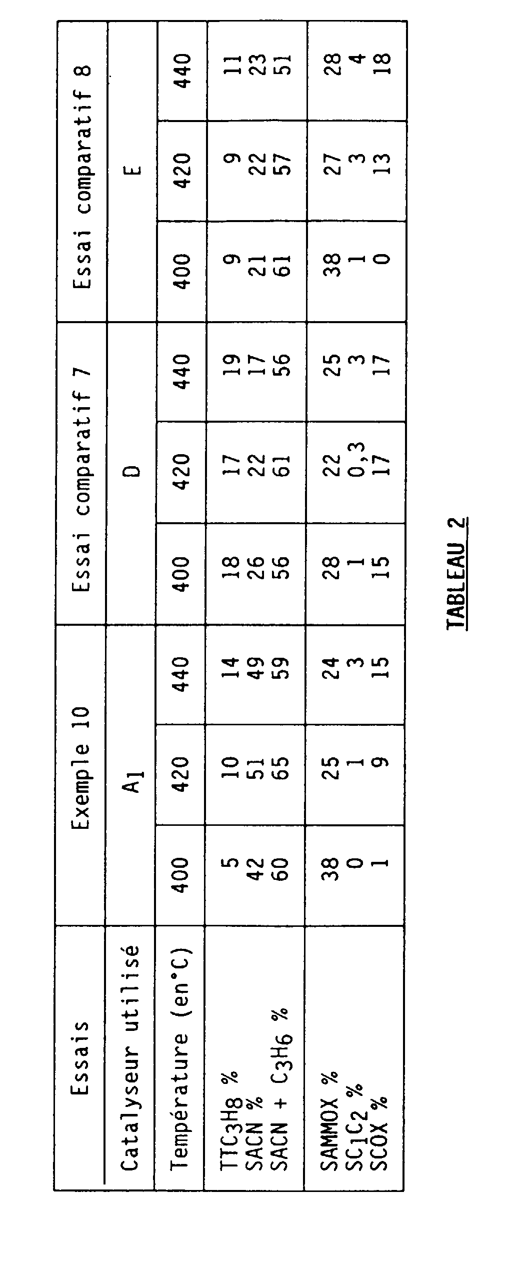

### EXEMPLE 11 ET ESSAI COMPARATIF 9

Mesure des performances des catalyseurs (A₁) et (E).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 7,5 %
   NH₃ = 15 %
   O₂ = 15 %
   H₂O = 20 %
   He = 42,5 %

Les conditions de température et les résultats obtenus sont rassemblés dans le tableau 3 ci-après.

**TABLEAU 3**

| Essais | Exemple 11 | | | | | Essai comparatif 9 | | | |
|---|---|---|---|---|---|---|---|---|---|
| Catalyseur utilisé | A₁ | | | | | E | | | |
| Température (en °C) | 400 | 420 | 440 | 460 | 480 | 400 | 420 | 440 | 460 |
| TTC₃H₈ % | 1 | 5 | 10 | 19 | 30 | 12 | 21 | 28 | 32 |
| SACN % | 6 | 53 | 67 | 78 | 83 | 41 | 56 | 62 | 61 |
| SACN + C₃H₆ % | 94 | 74 | 82 | 87 | 88 | 59 | 69 | 73 | 70 |
| SAMMOX % | 6 | 26 | 19 | 14 | 10 | 41 | 29 | 25 | 25 |
| SC₁C₂ % | 0 | 0 | 0 | 0 | 0 | 0 | 1 | 2 | 4 |
| SCOX % | 0 | 0 | 0 | 0 | 1 | 0 | 0 | 0 | 0 |

### EXEMPLE 12 ET ESSAI COMPARATIF 10

Mesure des performances des catalyseurs (B) et (C).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 25 %
   NH₃ = 10 %
   O₂ = 25 %
   H₂O = 20 %
   He = 20 %

Les conditions de température et les résultats obtenus sont rassemblés dans le tableau 4 ci-après.

**TABLEAU 4**

| Essais | Exemple 12 | | Essai comparatif 10 | |
|---|---|---|---|---|
| Catalyseur utilisé | B | | C | |
| Température (en°C) | 460 | 480 | 460 | 480 |
| TTC₃H₈ % | 10 | 14 | 36 | 36 |
| SACN % | 45 | 40 | 48 | 23 |
| SACN + C₃H₆ % | 73 | 67 | 54 | 61 |
| SAMMOX % | 23 | 22 | 36 | 23 |
| SC₁C₂ % | 2 | 3 | 1 | 1 |
| SCOX % | 2 | 8 | 9 | 14 |

### EXEMPLE 13 ET ESSAI COMPARATIF 11

Mesure des performances des catalyseurs (B) et (C).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 7,5 %
   NH₃ = 15 %
   O₂ = 15 %
   H₂O = 20 %
   He = 42,5 %

Les conditions de température et les résultats obtenus sont rassemblés dans le tableau 5 ci-après.

**TABLEAU 5**

| Essais | Exemple 13 | | Essai comparatif 11 | |
|---|---|---|---|---|
| Catalyseur utilisé | B | | C | |
| Température (en°C) | 460 | 480 | 460 | 480 |
| TTC₃H₈ % | 11 | 14 | 23 | 28 |
| SACN % | 46 | 53 | 38 | 47 |
| SACN + C₃H₆ % | 71 | 77 | 48 | 55 |
| SAMMOX % | 24 | 21 | 49 | 42 |
| SC₁C₂ % | 5 | 2 | 1 | 4 |
| SCOX % | 0 | 0 | 2 | 0 |

### EXEMPLE 14

Mesure des performances du catalyseur (A₂).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 48 %
   NH₃ = 9 %
   O₂ = 18 %
   H₂O = 20 %
   He = 5 %

Les conditions de température et les résultats obtenus sont rassemblés dans le tableau 6 ci-après.

**TABLEAU 6**

| | 460°C | 480°C |
|---|---|---|
| TTC₃H₈ % | 2 % | 3,5 % |
| SACN % | 17 % | 12 % |
| SACN + C₃H₆ % | 65 % | 65 % |
| SAMOX % | 33 % | 27 % |
| SC₁C₂ % | 2 % | 8 % |
| SCOX % | 0 | 0 |

### EXEMPLE 15 ET ESSAI COMPARATIF 12

Mesure des performances des catalyseurs (G) et (F).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 7,5 %
   NH₃ = 15 %
   O₂ = 15 %
   H₂O = 20 %
   He = 42,5 %

Les conditions de température et les résultats obtenus sont rassemblés dans le tableau 7 ci-après.

**TABLEAU 7**

| Essais | Exemple 15 | | | | Essai comparatif 12 | | | |
|---|---|---|---|---|---|---|---|---|
| Catalyseur utilisé | G | | | | F | | | |
| Température (en°C) | 360 | 380 | 400 | 440 | 360 | 380 | 400 | 440 |
| TTC₃H₈ % | 6 | 18 | 33 | 51 | 3 | 6 | 14 | 30 |
| SACN % | 13 | 32 | 38 | 53 | 3 | 5 | 8 | 8 |
| SACN + C₃H₆ % | 46 | 46 | 47 | 60 | 15 | 14 | 13 | 10 |
| SAMMOX % | 50 | 45 | 33 | 26 | 85 | 85 | 87 | 88 |
| SC₁C₂ % | 0 | 0 | 0 | 0 | 0 | 0 | 0 | 2 |
| SCOX % | 4 | 8 | 20 | 13 | 0 | 0 | 0 | 0 |

### EXEMPLE 16

Mesure des performances du catalyseur (G).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 20 %
   NH₃ = 20 %
   O₂ = 20 %
   H₂O = 20 %
   He = 20 %

Les conditions de température et les résultats obtenus sont rassemblés dans le tableau 8 ci-après.

**TABLEAU 8**

| | 360°C | 380°C | 400°C | 420°C |
|---|---|---|---|---|
| TTC₃H₈ % | 23 % | 26 % | 26 % | 26 % |
| SACN % | 34 % | 32 % | 25 % | 26 % |
| SACN + C₃H₆ % | 51 % | 52 % | 49 % | 51 % |
| SAMMOX % | 36 % | 36 % | 37 % | 34 % |
| SC₁C₂ % | 0,1 % | 0,3 % | 0,2 % | 0 |
| SCOX % | 13 % | 12 % | 14 % | 15 % |

### EXEMPLE 17 ET ESSAI COMPARATIF 13

Mesure des performances des catalyseurs (H) et (M).

Les conditions opératoires mises en oeuvre sont les suivantes
- Vitesse volumique horaire = 1000h⁻¹
- Pression totale = 1,3 bar
- Composition volumique du mélange réactionnel :
   C₃H₈ = 7,5 %
   NH₃ = 15 %
   O₂ = 15 %
   H₂O = 20 %
   He = 42,5 %

Les conditions de température et les résultats obtenus sont rassemblés dans le tableau 9 ci-après.

**TABLEAU 9**

| Essais | Exemple 17 | | | | | Essai comparatif 13 | | | |
|---|---|---|---|---|---|---|---|---|---|
| Catalyseur utilisé | H | | | | | M | | | |
| Température (en°C) | 380 | 400 | 420 | 440 | 460 | 400 | 420 | 440 | 460 |
| TTC₃H₈ % | 3 | 7 | 16 | 21 | 23 | 5 | 15 | 31 | 38 |
| SACN % | 47 | 73 | 85 | 90 | 81 | 34 | 66 | 74 | 66 |
| SACN + C₃H₆ % | 68 | 84 | 89 | 93 | 83 | 65 | 78 | 81 | 75 |
| SAMMOX % | 32 | 16 | 11 | 6 | 7 | 33 | 18 | 18 | 10 |
| SC₁C₂ % | 0 | 0 | 0 | 0 | 10 | 0 | 4 | 0 | 0 |
| SCOX % | 0 | 0 | 0 | 0 | 0 | 2 | 0 | 0 | 16 |

### EXEMPLE 18 ET ESSAI COMPARATIF 14

Mesure des performances des catalyseurs (H) et (M).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 48 %
   NH₃ = 9 %
   O₂ = 18 %
   H₂O = 20 %
   He = 5 %

Les conditions de température et les résultats obtenus sont rassemblés dans le tableau 10 ci-après.

**TABLEAU 10**

| Essais | Exemple 18 | | | Essai comparatif 14 | | |
|---|---|---|---|---|---|---|
| Catalyseur utilisé | H | | | M | | |
| Température (en°C) | 360 | 380 | 400 | 360 | 380 | 400 |
| TTC₃H₈ % | 3 | 6 | 10 | 4 | 10 | 12 |
| SACN % | 20 | 47 | 47 | 10 | 34 | 36 |
| SACN + C₃H₆ % | 37 | 63 | 58 | 43 | 60 | 63 |
| SAMMOX % | 63 | 37 | 30 | 56 | 39 | 36 |
| SC₁C₂ % | 0 | 0 | 1 | 0 | 0 | 0 |
| SCOX % | 0 | 0 | 10 | 0 | 0 | 0 |

### EXEMPLE 19

Mesure des performances du catalyseur (J).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 7,5 %
   NH₃ = 15 %
   O₂ = 15 %
   H₂O = 20 %
   He = 42,5 %

Les conditions de température et les résultats obtenus sont rassemblés dans le tableau 11 ci-après.

**TABLEAU 11**

| Température (en°C) | 380 | 400 | 420 | 440 | 460 |
|---|---|---|---|---|---|
| TTC₃H₈ % | 3 | 7 | 14 | 22 | 39 |
| SACN % | 23 | 48 | 80 | 89 | 82 |
| SACN + C₃H₆ % | 38 | 55 | 85 | 92 | 84 |
| SAMMOX % | 62 | 45 | 15 | 8 | 16 |
| SC₁C₂ % | 0 | 0 | 0 | 0 | 0 |
| SCOX % | 0 | 0 | 0 | 0 | 0 |

### EXEMPLES 20 ET 21

Mesure des performances du catalyseur (J).

Les conditions opératoires mises en oeuvre sont les suivantes :
- Vitesse volumique horaire = 1000h⁻¹
- Pression totale = 1,3 bar
- Composition volumique du mélange réactionnel :
   Exemple 20 :
   C₃H₈ = 20 %
   NH₃ = 20 %
   O₂ = 20 %
   H₂O = 20 %
   He = 20 %
   Exemple 21 :
   C₃H₈ = 48 %
   NH₃ = 9 %
   O₂ = 18 %
   H₂O = 20 %
   He = 5 %

Les conditions de température et les résultats obtenus sont rassemblés dans le tableau 12 ci-après.

**TABLEAU 12**

| | Exemple 20 | | | Exemple 21 | |
|---|---|---|---|---|---|
| Température (en°C) | 380 | 400 | 420 | 380 | 400 |
| TTC₃H₈ % | 3 | 5 | 9 | 9 | 9 |
| SACN % | 30 | 56 | 52 | 31 | 32 |
| SACN + C₃H₆ % | 41 | 65 | 59 | 42 | 44 |
| SAMMOX % | 54 | 35 | 41 | 58 | 26 |
| SC₁C₂ % | 5 | 0 | 0 | 0 | 1 |
| SCOX % | 0 | 0 | 0 | 0 | 28 |

### EXEMPLE 22

Mesure des performances du catalyseur (K).

Les conditions opératoires mises en oeuvre sont les suivantes
- Vitesse volumique horaire = 1000h⁻¹
- Pression totale = 1,3 bar
- Composition volumique du mélange réactionnel :
   C₃H₈ = 7,5 %
   NH₃ = 15 %
   O₂ = 15 %
   H₂O = 20 %
   He = 42,5 %

Les conditions de température et les résultats obtenus sont rassemblés dans le tableau 13 ci-après.

**TABLEAU 13**

| Température (en°C) | 360 | 380 | 400 | 420 | 440 | 460 |
|---|---|---|---|---|---|---|
| TTC₃H₈ % | 4 | 11 | 18 | 29 | 39 | 40 |
| SACN % | 31 | 56 | 75 | 84 | 77 | 71 |
| SACN + C₃H₆ % | 43 | 61 | 80 | 87 | 80 | 74 |
| SAMMOX % | 57 | 39 | 20 | 10 | 14 | 21 |
| SC₁C₂ % | 0 | 0 | 0 | 0 | 0 | 1 |
| SCOX % | 0 | 0 | 0 | 3 | 6 | 4 |

### EXEMPLE 23

Mesure des performances du catalyseur (K).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 20 %
   NH₃ = 20 %
   O₂ = 20 %
   H₂O = 20 %
   He = 20 %

Les conditions de température et les résultats obtenus sont rassemblés dans le tableau 14 ci-après.

**TABLEAU 14**

| Température (en°C) | 360 | 380 | 400 | 410 |
|---|---|---|---|---|
| TTC₃H₈ % | 3 | 7 | 21 | 23 |
| SACN % | 25 | 53 | 54 | 57 |
| SACN + C₃H₆ % | 39 | 65 | 60 | 65 |
| SAMMOX % | 61 | 35 | 40 | 35 |
| SC₁C₂ % | 0 | 0 | 0 | 0 |
| SCOX % | 0 | 0 | 0 | 0 |

### EXEMPLE 24

Mesure des performances du catalyseur (K).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 48 %
   NH₃ = 9 %
   O₂ = 18 %
   H₂O = 20 %
   He = 5 %

Les conditions de température et les résultats obtenus sont rassemblés dans le tableau 15 ci-après.

**TABLEAU 15**

| Température (en°C) | 360 | 380 | 390 | 400 | 410 |
|---|---|---|---|---|---|
| TTC₃H₈ % | 6 | 9 | 11 | 12 | 12 |
| SACN % | 19 | 28 | 32 | 42 | 46 |
| SACN + C₃H₆ % | 37 | 49 | 48 | 56 | 60 |
| SAMMOX % | 63 | 51 | 40 | 28 | 24 |
| SC₁C₂ % | 0 | 0 | 0 | 1 | 0 |
| SCOX % | 0 | 0 | 12 | 15 | 16 |

### EXEMPLE 25

Mesure des performances du catalyseur (L).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 7,5 %
   NH₃ = 15 %
   O₂ = 15 %
   H₂O = 20 %
   He = 42,5 %

Les conditions de température et les résultats obtenus sont rassemblés dans le tableau 16 ci-après.

**TABLEAU 16**

| Température (en°C) | 380 | 400 | 420 | 440 | 460 | 470 |
|---|---|---|---|---|---|---|
| TTC₃H₈ % | 1 | 3 | 5 | 7 | 16 | 29 |
| SACN % | 18 | 33 | 53 | 70 | 84 | 90 |
| SACN + C₃H₆ % | 18 | 42 | 61 | 76 | 86 | 91 |
| SAMMOX % | 82 | 58 | 39 | 24 | 14 | 7 |
| SC₁C₂ % | 0 | 0 | 0 | 0 | 0 | 2 |
| SCOX % | 0 | 0 | 0 | 0 | 0 | 0 |

### EXEMPLE 26

Mesure des performances du catalyseur (L).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 20 %
   NH₃ = 20 %
   O₂ = 20 %
   H₂O = 20 %
   He = 20 %

Les conditions de température et les résultats obtenus sont rassemblés dans le tableau 17 ci-après.

**TABLEAU 17**

| Température (en°C) | 380 | 400 | 420 | 440 |
|---|---|---|---|---|
| TTC₃H₈ % | 2 | 4 | 8 | 13 |
| SACN % | 14 | 28 | 50 | 72 |
| SACN + C₃H₆ % | 17 | 31 | 53 | 74 |
| SAMMOX % | 83 | 69 | 47 | 26 |
| SC₁C₂ % | 0 | 0 | 0 | 0 |
| SCOX % | 0 | 0 | 0 | 0 |

### EXEMPLE 27

Mesure des performances du catalyseur (L).

Les conditions opératoires mises en oeuvre sont les suivantes :
- Vitesse volumique horaire = 1000h⁻¹
- Pression totale = 1,3 bar
- Composition volumique du mélange réactionnel :
   C₃H₈ = 48 %
   NH₃ = 9 %
   O₂ = 18 %
   H₂O = 20 %
   He = 5 %

Les conditions de température et les résultats obtenus sont rassemblés dans le tableau 18 ci-après.

**TABLEAU 18**

| Température (en°C) | 380 | 400 | 420 |
|---|---|---|---|
| TTC₃H₈ % | 3 | 5 | 11 |
| SACN % | 18 | 40 | 68 |
| SACN + C₃H₆ % | 25 | 47 | 73 |
| SAMMOX % | 75 | 53 | 26 |
| SC₁C₂ % | 0 | 0 | 1 |
| SCOX % | 0 | 0 | 0 |

## Revendications

1. Procédé d'ammoxydation d'alcanes en phase vapeur en présence d'un catalyseur solide comportant au moins une phase active, caractérisé en ce que la dite phase active répond à la formule empirique suivante (I) :
V Sb ₐ Bi_{b} Ox (I)
ou à la formule empirique suivante (II)
V Sbₐ Bi_{b} M_{c} Oₓ (II)
dans lesquelles :
- a représente un nombre entier ou fractionnaire égal ou supérieur à 1,
- b représente un nombre entier ou fractionnaire égal ou supérieur à 0,01,
- M représente un atome de fer et/ou de gallium et/ou d'indium,
- c représente un nombre entier ou fractionnaire égal ou supérieur à 0,1,
- x représente un nombre entier ou fractionnaire déterminé par le degré d'oxydation des autres éléments de la phase active.

2. Procédé selon la revendication 1, caractérisé en ce que ladite phase active répond à la formule (I) ou à la formule (II) dans lesquelles :
- le symbole a représente un nombre entier ou fractionnaire jusqu'à 20, compris de préférence entre 1 et 10,
- le symbole b représente un nombre entier ou fractionnaire jusqu'à 20, compris de préférence entre 0,1 et 10,
- le symbole c représente un nombre entier ou fractionnaire jusqu'à 20, compris de préférence entre 0,5 et 10.

3. Procédé selon l'une des revendications 1 ou 2, caractérisé en ce que l'alcane est le propane.

4. Procédé selon l'une des revendications 1 à 3, caractérisé en ce que la réaction est conduite en présence de vapeur d'eau.

5. Procédé selon l'une des revendications 1 à 4, caractérisé en ce que la température de réaction est comprise entre 300°C et 550°C et de préférence entre 400°C et 500°C.

6. Procédé selon l'une des revendications 1 à 5, caractérisé en ce que la pression totale est comprise entre 1 et 6 bar.

7. Procédé selon l'une des revendications 1 à 6, caractérisé en ce que la vitesse volumique horaire est comprise entre 100 et 36 000 h⁻¹.

8. Procédé selon l'une des revendications 1 à 7, caractérisé en ce que dans le gaz réactif (mélange d'hydrocarbure saturé, d'ammoniac et d'oxygène) la teneur en hydrocarbure saturé est comprise entre 5 et 70 %, la teneur en ammoniac est comprise entre 3 et 50 % et la teneur en oxygène est comprise entre 3 et 45 %.

9. Procédé selon l'une des revendications 1 à 8, caractérisé en ce que la composition du mélange gazeux est en dehors du domaine d'explosivité.

10. Procédé selon l'une des revendications 1 à 9, caractérisé en ce que le catalyseur solide renferme, outre ladite phase active, au moins un oxyde minéral.

11. Procédé selon la revendication 10, caractérisé en ce que l'oxyde minéral est choisi parmi l'alumine, la silice, la silice-alumine, la zircone, la cérine, la magnésie, l'oxyde de titane, l'oxyde de niobium et leurs mélanges.

12. Procédé selon l'une des revendications 1 à 9, caractérisé en ce que le catalyseur solide renferme, outre ladite phase active, un support inerte ou un substrat céramique ou métallique de type monolithique.

13. Procédé selon l'une des revendications 10 ou 11, caractérisé en ce que le catalyseur solide renferme également un support inerte ou un substrat céramique ou métallique de type monolithique.

14. Procédé selon l'une des revendications 10 ou 11, caractérisé en ce que la phase active du catalyseur représente de 5 % à 100 % en poids du poids dudit catalyseur et de préférence de 10 % à 50 % en poids par poids.

15. Procédé selon l'une des revendications 12 ou 13, caractérisé en ce que la phase active ou la phase catalytique représente de 1 % à 50 % en poids du poids du catalyseur et de préférence de 5 % à 35 % en poids par poids.

## Patentansprüche

1. Verfahren zur Ammoxidation von Alkanen in der Dampfphase und in Anwesenheit eines festen Katalysators, der mindestens eine aktive Phase umfaßt, dadurch gekennzeichnet, daß die genannte aktive Phase der folgenden empirischen Formel (I):
V Sbₐ Si_{b} Oₓ (I)
oder der folgenden empirischen Formel (II):
V Sbₐ Bi_{b} M_{c} Oₓ (II)
entspricht, worin
- a eine ganze oder gebrochene Zahl von gleich oder höher als 1 darstellt,
- b eine ganze oder gebrochene Zahl von gleich oder höher als 0,01 darstellt,
- M ein Atom von Eisen und/oder Gallium und/oder Indium bedeutet,
- c eine ganze oder gebrochene Zahl von gleich oder höher als 0,1 darstellt,
- x eine ganze oder gebrochene Zahl bedeutet, die durch den Oxidationsgrad der anderen Elemente der aktiven Phase bestimmt wird.

2. Verfahren nach Amspruch 1, dadurch gekennzeichnet, daß die genannte aktive Phase der Formel (I) oder der Formel (II) entspricht, worin
- das Symbol a eine ganze oder gebrochene Zahl von bis zu 20, vorzugsweise zwischen 1 und 10 darstellt,
- das Symbol b eine ganze oder gebrochene Zahl von bis zu 20, vorzugsweise zwischen 0,1 und 10 darstellt,
- das Symbol c eine ganze oder gebrochene Zahl von bis zu 20, vorzugsweise zwischen 0,5 und 10 darstellt.

3. Verfahren nach einem der Ansprüche 1 oder 2, dadurch gekennzeichnet, daß das Alkan Propan ist.

4. Verfahren nach einem der Ansprüche 1 bis 3, dadurch gekennzeichnet, daß die Reaktion in Anwesenheit von Wasserdampf durchgeführt wird.

5. Verfahren nach einem der Ansprüche 1 bis 4, dadurch gekennzeichnet, daß die Temperatur der Reaktion zwischen 300 °C und 550 °C, vorzugsweise zwischen 400 °C und 500 °C liegt.

6. Verfahren nach einem der Ansprüche 1 bis 5, dadurch gekennzeichnet, daß der Gesamtdruck zwischen 1 und 6 bar liegt.

7. Verfahren nach einem der Ansprüche 1 bis 6, dadurch gekennzeichnet, daß die Stundenvolumen-Geschwindigkeit zwischen 100 und 36000 h⁻¹ liegt.

8. Verfahren nach einem der Ansprüche 1 bis 7, dadurch gekennzeichnet, daß in dem Reaktionsgas (Mischung von gesättigtem Kohlenwasserstoff, Ammoniak und Sauerstoff) der Gehalt an gesättigtem Kohlenwasserstoff zwischen 5 % und 70 %, der Gehalt an Ammoniak zwischen 3 % und 50 % und der Gehalt an Sauerstoff zwischen 3 % und 45 % liegt.

9. Verfahren nach einem der Ansprüche 1 bis 8, dadurch gekennzeichnet, daß die Zusammensetzung der Gasmischung außerhalb des Bereiches der Explosionsfähigkeit liegt.

10. Verfahren nach einem der Ansprüche 1 bis 9, dadurch gekennzeichnet, daß der feste Katalysator außer der genannten aktiven Phase mindestens ein mineralisches Oxid umfaßt.

11. Verfahren nach Anspruch 10, dadurch gekennzeichnet, daß das mineralische Oxid unter Aluminiumoxid, Siliciumdioxid, Silicium-Aluminiumoxid, Zirkoniumdioxid, Ceroxid, Magnesiumoxid, Titanoxid, Niobiumoxid und ihren Mischungen ausgewählt wird.

12. Verfahren nach einem der Ansprüche 1 bis 9, dadurch gekennzeichnet, daß der feste Katalysator außer der genannten aktiven Phase einen inerten Träger oder ein keramisches oder metallisches Substrat vom monolithischen Typ umfaßt.

13. Verfahren nach einem der Ansprüche 10 oder 11, dadurch gekennzeichnet, daß der feste Katalysator ebenfalls einen inerten Träger oder ein keramisches oder metallisches Substrat vom monolithischen Typ umfaßt.

14. Verfahren nach einem der Ansprüche 10 oder 11, dadurch gekennzeichnet, daß die aktive Phase des Katalysators 5 Gew.-% bis 100 Gew.-% des Gewichtes des genannten Katalysators und vorzugsweise 10 Gew.-% bis 50 Gew.-% darstellt.

15. Verfahren nach einem der Ansprüche 12 oder 13, dadurch gekennzeichnet, daß die aktive Phase oder die katalytische Phase 1 Gew.-% bis 50 Gew.-% des Gewichtes des Katalysators und vorzuasweise 5 Gew.-% bis 35 Gew.-% darstellt.

## Claims

1. Process for the ammoxidation of alkanes in the vapour phase in the presence of a solid catalyst comprising at least one active phase, characterized in that the said active phase corresponds to the following empirical formula (I):
VSbₐBi_{b}Oₓ (I)
or to the following empirical formula (II):
VSbₐBi_{b}M_{c}Oₓ (II)
in which formulae:
- a represents a whole or fractional number equal to or greater than 1,
- b represents a whole or fractional number equal to or greater than 0.01,
- M represents an iron and/or gallium and/or indium atom,
- c represents a whole or fractional number equal to or greater than 0.1,
- x represents a whole or fractional number specified by the oxidation number of the other elements of the active phase.

2. Process according to claim 1, characterized in that the said active phase corresponds to the formula (I) or to the formula (II) in which formulae:
- the symbol a represents a whole or fractional number up to 20, preferably of between 1 and 10,
- the symbol b represents a whole or fractional number up to 20, preferably of between 0.1 and 10,
- the symbol c represents a whole or fractional number up to 20, preferably of between 0.5 and 10.

3. Process according to either of claims 1 or 2, characterized in that the alkane is propane.

4. Process according to one of claims 1 to 3, characterized in that the reaction is carried out in the presence of steam.

5. Process according to one of claims 1 to 4, characterized in that the reaction temperature is between 300°C and 550°C and preferably between 400°C and 500°C.

6. Process according to one of claims 1 to 5, characterized in that the total pressure is between 1 and 6 bar.

7. Process according to one of claims 1 to 6, characterized in that the hourly volume rate is between 100 and 36,000 h⁻¹.

8. Process according to one of claims 1 to 7, characterized in that, in the reactive gas (mixture of saturated hydrocarbon, ammonia and oxygen), the content of saturated hydrocarbon is between 5 and 70 %, the content of ammonia is between 3 and 50 % and the content of oxygen is between 3 and 45 %.

9. Process according to one of claims 1 to 8, characterized in that the composition of the gaseous mixture is outside the explosive region.

10. Process according to one of claims 1 to 9, characterized in that the solid catalyst contains, besides the said active phase, at least one inorganic oxide.

11. Process according to claim 10, characterized in that the inorganic oxide is chosen from alumina, silica, silica/alumina, zirconia, cerite, magnesia, titanium oxide, niobium oxide and their mixtures.

12. Process according to one of claims 1 to 9, characterized in that the solid catalyst contains, besides the said active phase, an inert support or a ceramic or metal substrate of monolithic type.

13. Process according to either of claims 10 or 11, characterized in that the solid catalyst also contains an inert support or a ceramic or metal substrate of monolithic type.

14. Process according to either of claims 10 or 11, characterized in that the active phase of the catalyst represents from 5 % to 100 % by weight of the weight of the said catalyst and preferably from 10 % to 50 % weight/weight.

15. Process according to either of claims 12 or 13, characterized in that the active phase or the catalytic phase represents from 1 % to 50 % by weight of the weight of the catalyst and preferably from 5 % to 35 % weight/weight.
